(19) Europäisches Patentamt
European Patent Office
Office européen des brevets

(11) **EP 3 763 293 A1**

(12) **EUROPEAN PATENT APPLICATION**

(43) Date of publication:
**13.01.2021 Bulletin 2021/02**

(51) Int Cl.:
**A61B 6/10** *(2006.01)*　　　**A61B 6/03** *(2006.01)*
**A61B 6/00** *(2006.01)*　　　**A61B 6/06** *(2006.01)*

(21) Application number: **20185090.6**

(22) Date of filing: **09.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.07.2019 US 201962871803 P**

(71) Applicant: **Mistretta Medical LLC**
**Madison, Wisconsin 53717 (US)**

(72) Inventors:
• **Mistretta, Charles A.**
  **Madison, Wisconsin 53704 (US)**
• **Wagner, Martin G.**
  **Madison, Wisconsin 53705 (US)**

(74) Representative: **Potter Clarkson**
**The Belgrave Centre**
**Talbot Street**
**Nottingham NG1 5GG (GB)**

(54) **SYSTEM FOR LOW DOSE CT FLUOROSCOPY VIA APERTURE CONTROL**

(57)　An X-ray filter can include a curved structure for positioning around a computed tomography (CT) bore of a CT scanner. The curved structure can be formed of a metal capable of blocking X-rays emitted by an X-ray source of the CT scanner. The curved structure can include two or more apertures. Each aperture of the two or more apertures can allow X-rays emitted by the X-ray source to enter the CT bore when the X-ray source is aligned with the aperture. The X-ray filter can include an opening, arranged opposite to the two or more apertures across the CT bore, for exposing an X-ray detector of the CT scanner to X-rays emitted by the X-ray source and entering the CT bore through at least one of the two or more apertures. The CT scanner can include the X-ray filter.

FIG. 1

EP 3 763 293 A1

## Description

## CROSS-REFERENCES TO RELATED APPLICATIONS

This application claims the benefit of and priority to the U.S. Provisional Application No. 62/871,803 filed on July 9, 2019, which is incorporated herein by reference in its entirety.

## BACKGROUND OF THE DISCLOSURE

CT-guided percutaneous interventions represent an integral component of clinical interventional radiology practices. Many important diagnostic and therapeutic procedures are performed using CT guidance, including biopsies, placement of drainage tubes and catheters, tumor ablation, and musculoskeletal interventions, among others. Examples of such diagnostic and therapeutic procedures include CT-guided biopsy and tumor ablation for the diagnosis and treatment of patients with many different types of cancer, the second leading cause of death in the United States. An estimated ~1.7 million people were diagnosed with cancer in the US in 2016. The vast majority will have undergone biopsy for diagnosis. From 1997 to 2008 the number of biopsies performed by radiologists increased with a compound annual growth rate of 8% and over 58,000 CT-guided lung biopsies were performed in 2004.

CT guided CT interventions allow for determining appropriate puncture sites, direction of insertion of a needle (or interventional device), and placement of the interventional device after each adjustment. CT guided interventions can be performed using conventional CT (CCT) or CT fluoroscopy (CTF). The CCT involves a full dose axial or helical scan with the resulting scan having limited z-axis coverage. For CTF, however, CT scanners perform helical scans and can acquire projections associated with up to three z-axis scan locations.

## SUMMARY

At least one aspect is directed to an X-ray filter. The X-ray filter can include a curved structure for positioning around a computed tomography (CT) bore of a CT scanner. The curved structure can be formed of a metal capable of blocking X-rays emitted by an X-ray source of the CT scanner. The curved structure can include two or more apertures. Each aperture of the two or more apertures can allow X-rays emitted by the X-ray source to enter the CT bore when the X-ray source is aligned with the aperture. The X-ray filter can include an opening, arranged opposite to the two or more apertures across the CT bore, for exposing an X-ray detector of the CT scanner to X-rays emitted by the X-ray source and entering the CT bore through at least one of the two or more apertures.

At least one other aspect is directed to a computed tomography (CT) scanner. The CT scanner can include a CT bore, a rotating gantry including an X-ray source and an X-ray detector positioned opposite to one another across the CT bore, one or more processors, and an X-ray filter. The X-ray filter can include a curved structure for positioning around the CT bore. The curved structure can be formed of a metal capable of blocking X-rays emitted by the X-ray source, and can include two or more apertures. Each aperture of the two or more apertures can allow X-rays emitted by the X-ray source to enter the CT bore when the X-ray source is aligned with the first aperture. The X-ray filter can include an opening, arranged opposite to the two or more apertures across the CT bore, for exposing the X-ray detector to X-rays emitted by the X-ray source and entering the CT bore through at least one of the two or more apertures.

At least one other aspect is directed to an X-ray filter. The X-ray filter can include a curved structure for positioning around a computed tomography (CT) bore of a CT scanner. The curved structure can be formed of a metal capable of blocking X-rays emitted by an X-ray source of the CT scanner, and can include a copper-filled aperture for allowing a portion of X-rays emitted by the X-ray source to enter the CT bore when the X-ray source is aligned with the copper-filled aperture. The copper-filled aperture can have a breadth such that the portion of X-rays entering the CT bore through the aperture, when the X-ray source is aligned with a center of the aperture, excites the whole CT detector. The X-ray filter can include an opening, arranged opposite to the aperture across the CT bore, for exposing an X-ray detector of the CT scanner to the portion of X-rays emitted by the X-ray source and entering the CT bore through the aperture.

At least one other aspect is directed to a computed tomography (CT) scanner. The CT scanner can include a CT bore, a rotating gantry including an X-ray source and an X-ray detector positioned opposite to one another across the CT bore, one or more processors, and an X-ray filter. The X-ray filter can include a curved structure for positioning around the CT bore. The curved structure can be formed of a metal capable of blocking X-rays emitted by the X-ray source, and can include a coper-filled aperture for allowing a portion of X-rays emitted by the X-ray source to enter the CT bore when the X-ray source is aligned with the copper-filled aperture. The copper-filled aperture can have a breadth such that the portion of X-rays entering the CT bore through the aperture, when the X-ray source is aligned with a center of the aperture, excites the whole CT detector. The X-ray filter can include an opening, arranged opposite to the copper-filled aperture across the CT bore for exposing the X-ray detector to the portion of X-rays emitted by the X-ray source and entering the CT bore through at least one of the two or more apertures.

## BRIEF DESCRIPTION OF THE DRAWINGS

FIG. 1 is a diagram illustrating one embodiment of a rotational X-ray system, according to inventive concepts of this disclosure;

FIGS. 2A and 2B show diagrams illustrating an example X-ray filter, according to inventive concepts of this disclosure;

FIG. 3 shows an example illustration of multiple partial exposures of an X-ray detector array assembly to X-ray beams passing through a single aperture of an X-ray filter, according to inventive concepts of this disclosure;

FIGS. 4A and 4B show other example X-ray filters, according to inventive concepts of this disclosure;

FIG. 5 shows simulation results illustrating effect of integration of, or combining multiple projection images on image quality, according to inventive concepts of this disclosure;

FIG. 6 shows plots illustrating audio signals recorded by a piezo microphone for various gantry revolution speeds;

FIG. 7 shows a diagram illustrating a method of determining the position, orientation and/or shape of the interventional device in the 3D space, according to inventive concepts of this disclosure;

FIG. 8 shows a comparison between conventional CTF reconstructions of the interventional device and the anatomical region and reconstructions using only two projections as discussed above;

FIG. 9 shows an experimental setup including a preliminary X-ray filter;

FIG. 10 shows an overview of a model-based motion compensation system, according to inventive concepts of this disclosure; and

FIG. 11 shows experimental results with and without motion compensation, where a 3D volume of an anatomical region is overlaid with the conventional reconstruction of the interventional device at a random respiratory state.

## DETAILED DESCRIPTION

There are two main approaches for performing CT-guided interventions. A first approach is CCT, which involves performing a series of conventional (or full dose) axial or helical CT acquisitions. The maximum z-axis coverage of CCT acquisitions is usually between 4 cm and 16 cm. CCT also involves performing a pre-procedural diagnostic quality scan, prior to inserting a needle (or other interventional device) in an anatomical region, to define a target region or a region of interest (ROI) as well as a point of entry and anticipated path of the interventional device. A physician can insert the interventional device in the anatomical region, and then repeatedly advance and/or adjust the interventional device according to a series of steps. After each adjustment or advancement of the interventional device, a CT scanner can perform a full dose CT acquisition to determine the current position of the interventional device, and the next adjustment of interventional device if any.

The full dose (or CCT) scanning presents serious health risks for operators (e.g., physicians and/or health care practitioners) involved in CT-guided intervention procedures due to the potential cumulative radiation dosage acquired by these individuals overtime. To mitigate such risks, operators make adjustments to the position of the interventional device and then step out of the room during the following CT acquisition. The steps of adjusting the interventional device, leaving the room, performing a CT image acquisition, and examining the acquired CT image to decide on the next adjustment, if any, are repeated until the interventional device reaches the target region or ROI. This approach makes the CT-guided intervention procedure time consuming, inconvenient and tedious for both the operator and the patient. For instance, the longer is the CT-guided intervention procedure, the larger may be the sedation dose used and the more likely the patient will move adding more complexity and inaccuracy to the process of tracking the placement of the interventional device after successive adjustments. Furthermore, the radiation dose for the patient is relatively high due to the repeated CT acquisitions.

To speed-up the CT-guided intervention process or reduce the number of adjustments, physicians may perform fairly aggressive adjustments of the interventional device (e.g., relatively large advancement of the interventional device at each step). However, in such case, the adjustments are performed without real-time feedback and can be less precise (e.g., compared to relatively smaller adjustments). The fairly aggressive adjustments can result in unwanted punctures of blood vessels, the heart, lungs or other organs. Incorrectly placed interventional devices (or needles) or repeated passes of the interventional device can also increase the risk for pneumothorax, bleeding and tumor seeding.

In contrast, CTF enables near real-time image guidance as well as "step-and-shoot" imaging at a lower dose using lesser tube currents and a limited axial field of view. CTF is generally performed at the lowest possible milliamperage (mA) to visualize the target, critical anatomy, and the interventional device in an attempt to decrease radiation exposure to the patient and operator(s). For instance, amperage can be approximately 20 mA in the chest where most targets are high contrast as compared with the background because of surrounding air while much higher doses are used for the abdomen due to lower contrast differences between the target and background tissue. In CTF, CT scanners generate images as three thick slices (2.5 mm) having considerably lower image quality and less z-axis coverage than CCT. Furthermore, both CCT and CTF images suffer from metal artifacts around a metal interventional device. Metal artifacts degrade the image quality especially around the interventional device. The low image quality and reduced field of view can make it difficult to visualize the target and localize the interventional device, which is often outside of the field of view.

Also, while radiation exposure for patients and operators is less with CTF when compared with CCT, patients and operators are still exposed to a considerable amount of radiation. Specifically, the physician and respective staff generally stay in the room during CTF acquisitions making them exposed to more radiation. For instance, typical median effective doses for patients are approximately 19.7 mSv, while for physicians and staff a whole-body dose (dose equivalent at 1 cm tissue depth) can be in the range of 0.007-0.048 mSv. On the other hand, CTF significantly reduces procedure time when compared to CCT as CTF projections can be acquired either continuously or in a series of intermittent acquisitions between adjustments of the interventional device the physician and staff remain in the operating room.

Other approaches for monitoring the motion of interventional devices can include electromagnetic tracking and/or optical tracking. For electromagnetic tracking, the position and orientation at the device tip can be determined using an electromagnetic (EM) field generator and sensor built into the interventional device. However, one drawback of electromagnetic tracking systems is that they do not produce images, and as such, they do not provide the actual location of the needle relative to the ROI in real-time. Additionally, EM systems require the purchase of separate capital equipment and disposables, have very primitive patient motion tracking capability based on external markers, and do not reconstruct device shape properties such as the curvature of the needle. For optical tracking, a single camera or stereoscopic camera setup can be used to track device position and orientation based on the external part of the device. Optical tracking techniques can be limited in that they assume a known device length and shape, which can be inaccurate in cases where the device is deformed during the procedure. Patient motion can be tracked using optical tracking techniques, but this is based only on external features, which might not account for internal organ motion or deformation. Ultrasound is another modality which is commonly used to guide percutaneous interventions, including biopsy. However, ultrasound is heavily dependent on operator skill, and requires an adequate sonographic or acoustic window. The latter limits its utility in certain body regions (e.g., in air-filled lungs or deep in the abdomen or pelvis behind bowel gas) or for certain body types (e.g., obese patients).

In the current disclosure, the clinical need for a CT-guided techniques that can be used throughout the body, provide high quality images without metal artifacts, and use only a small fraction of the X-ray radiation dose required for CCT or CTF is addressed. The driving idea is that as little as two projection images can be sufficient to reconstruct interventional devices such as percutaneous needles as illustrated, which could be used to considerably reduce radiation exposure. In order to achieve the proposed dose reduction, X-ray image acquisition is limited to selected view angles and relatively short X-ray pulses per view angle. While this could be achieved on a CT platform using pulsed X-ray tubes, which are turned on only for specific angles, many clinical CT systems use continuous X-ray exposure during the gantry rotation. , Pulsed tubes are commonly used in other applications such as micro-CT and X-ray angiography systems. Existing dual source CT scanners would be suitable to acquire X-ray images from only two views without rotating the gantry, but software modifications would still be required to implement this mode, and such a solution would be limited to dual-source CT scanners.

In current disclosure, aperture-based X-ray filters can be employed to various current clinical CT platforms without system modification. An X-ray filter can be placed inside the CT bore to block X-rays in all but few selected view angles. The use of X-ray filters can allow for accurate reconstruction and localization using as little as two projection images. Using as little as two projection images can significantly reduce X-ray radiation dose for patients and medical staff. Also, motion compensation techniques can be employed to compensate for patient motion during interventional procedures.

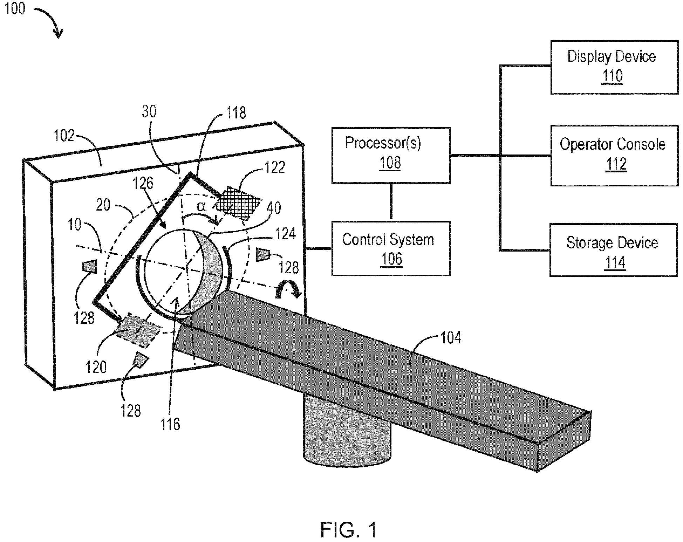

Referring to FIG. 1, a diagram illustrating one embodiment of a rotational X-ray system 100 is shown, according to inventive concepts of this disclosure. In brief overview, and by way of a non-limiting example, the rotational X-ray system 100 can include a CT scanner 102, a patient support 104 for accommodating a patient or subject, a control system 106, one or more processors 108, a display device 110, an operator console 112, and/or a storage device 114.

The CT scanner 102 can include a CT bore 116, a gantry 118 mechanically coupled to an X-ray source assembly 120 (also referred to as X-ray source) and an X-ray detector array assembly 122 (also referred to as X-ray detector), and an X-ray filter 124 positioned around or within the CT bore 116. The CT scanner 102 can include a plurality of microphones (or audio sensors) 128 for recording audio signals associated with movement of the X-ray source assembly 120 and/or movement of the X-ray detector array assembly 122. The control system 106 or the one or more processors 108 can use the recorded audio signals to track positions of the X-ray source assembly 120 and/or positions of the X-ray detector array assembly 122 as the gantry rotates. The gantry 118 can rotate around a horizontal axis 10 of the CT bore 116 while maintaining the source assembly 120 and the detector array assembly 122 opposite to one another across the CT bore 116. As the gantry 118 rotates around the axis 10, the X-ray source assembly 120 and the X-ray detector array assembly 122 can move along a path 20 around the CT bore 116 while facing each other. At any point in time, the X-ray source assembly 120 and the X-ray detector array assembly 122 can be facing each other along a diameter of the CT bore 116. X-rays emitted by the X-ray source assembly 120 can travel across the CT bore 116 towards the X-ray detector array assembly 122. While the gantry 118 is show to have a C-shape in FIG. 1, other possible shapes are contemplated by the current disclosure. The gantry 118 can be mechanically coupled to a motor (not shown in FIG. 1) to cause rotation of the gantry 118 when actuated.

The patient support 104 can slide toward and away from the CT scanner 102. In particular, the patient support 104 can slide into, and out of, the CT bore 116. When the patient support 104 is inside the CT bore 116, X-rays emitted by the source assembly 120 can penetrate at least an anatomical region of the patient lying on the patient support 104 before reaching the X-ray detector array assembly 122. As the gantry 118 rotates, the X-ray detector assembly 122 can acquire a plurality of X-ray projection images (or X-ray projections) at various orientation angles of the gantry 118. The X-ray projection images acquired at different orientation angles of the gantry 118 provide different views of the anatomical region.

The rotations of the gantry 118 around the axis 10 can enable the X-ray source assembly 120 and the X-ray detector array assembly 122 to be oriented in different positions and orientation angles (e.g., angle α between a reference axis 30 of the CT bore and an axis 40 of the X-ray beam emitted by the X-ray source assembly 120) around the patient disposed on the patient support 104, while enabling a physician to perform procedures on the patient. The X-ray source assembly 120 can emit an X-ray beam of directed at X-ray detector array assembly 122. Both assemblies 120 and 122 can be aligned and directed inward to the axis 10 to face each other across the CT bore 116. The longitudinal axis 40 of the emitted X-ray beam can pass through the center of the CT bore 116. Rotating the gantry 118 leads to a rotation the emitted X-ray beam (or the respective axis 40) around the axis 10 during the acquisition of X-ray data (or X-ray projection images) from a subject (e.g., patient) lying on the patient support 104.

The X-ray beam emitted by the X-ray source assembly 120 can impinge, e.g., after passing through the subject, on the X-ray detector array assembly 122. The X-ray detector array assembly 122 can include a two-dimensional array of detector elements. Each detector element produces an electrical signal that represents the intensity of an impinging X-ray and hence the attenuation of the X-ray as it passes through the subject (or a respective anatomical region). The control system 106 can cause the gantry 118 to rotate around the axis 10 (e.g., by actuating the motor), and/or can cause the X-ray source assembly 120 to emit X-ray beams while rotating around the CT bore 116. The control system 106 can cause the X-ray source assembly 120 to emit X-ray beams as it rotates around the CT bore 116. The control system 106 can cause the X-ray source assembly 120 to emit X-ray beams continuously along the whole path 20. The control system 106 can cause the X-ray source assembly 120 to emit X-ray beams according to a short scan scheme, where the X-ray source assembly 120 emits X-rays continuously along a portion of the path 20. The control system 106 can cause the X-ray source assembly 120 to emit X-ray beams according to a predefined pulsing scheme, where the X-ray source assembly 120 emits X-ray pulses according to a predefined timing scheme.

The X-ray detector array assembly 122 can be configured to acquire a predefined maximum number of X-ray projections (or projection images) per unit time (e.g., per second). Given a rotation speed of the gantry 118, the X-ray detector array assembly 122 can generate a predefined number of X-ray projections per gantry revolution. Even if the X-ray source assembly 120 is emitting X-rays continuously throughout the whole path 20, the X-ray detector array assembly 122 cannot generate more than the predefined maximum number of X-ray projections per unit time (or the predefined maximum number of X-ray projections per gantry revolution).

The X-ray filter 124 can include a curved structure for positioning around or within the CT bore 116. The curved structure can be formed of a metal (or other material) capable of blocking X-rays emitted by an X-ray source assembly 120. The curved structure can have a circular shape or a portion thereof, an elliptical shape or a portion thereof, a hyperbolic shape, or other curved shape. For instance, the curved structure can form a portion of a ring. The curved structure can include an opening or a gap 126 (e.g., the missing portion of the curved structure to form a full ring or full cylinder) for letting X-rays through. The curved structure can include one or more apertures (not shown in FIG. 1) arranged within the curved structure. As discussed in further detail below, the X-ray filter 124 can allow for significant reduction in exposure to radiation by the patient and/or medical staff performing the interventional procedure on the patient. The X-ray filter 124 can be integrated in (and a part of) the CT scanner 102, or can be a separate piece capable of being mechanically coupled to (and detached from) the CT scanner 102. For instance, the X-ray filter 124 can be placed inside (and removed from) the CT bore 116.

The control system 106 can control the rotation of the gantry 118 and the operation of the X-ray source assembly 120. The control system 106 can include an X-ray controller (not shown in FIG. 1) that can provide power and/or timing signals to the X-ray source assembly 120. In the case of X-ray pulsing, the X-ray controller can provide a pulse train to cause the X-ray source assembly 120 to emit X-ray beams timed by (or actuate the X-ray source assembly 120 during each pulse of) the pulse train. In the case of a short scan acquisition scheme, the X-ray controller can actuate the X-ray source assembly during a predefined portion of each gantry revolution and switch off (or deactivate) the X-ray source assembly 120 during the rest of the gantry revolution. For a continuous scanning scheme, the X-ray controller can actuate the X-ray source assembly throughout one or more complete gantry revolutions. The control system 106 can include a data acquisition system (not shown in FIG. 1) that can sample data from the detector elements of the X-ray detector array assembly 122, and pass the data to the one or more processors 108. The control system 106 can also include a gantry motor controller (not shown in FIG. 1), for actuating the motor to cause the gantry 118 to rotate around the axis 10. The gantry motor controller can receive motion commands from the one or more processors 108 and provide power to the gantry 118 responsive to such commands.

The gantry 118, the X-ray source assembly 120, the X-ray detector array assembly 122, the patient support 104, the control system 106, or any combination thereof can be viewed as being part of the CT scanner device 102. The one or more processors 108, the display device 110, the operator console 112, the storage device 114, or any combination thereof can be integrated within the CT scanner 102, a computing device communicatively coupled to the CT scanner, or a combination thereof. The one or more processors 108 can execute computer code instructions to cause CT data acquisition, generate CT images, cause display of generated CT images, store generated images or CT projection data in the storage device 114, or a combination thereof. The computer code instructions can include executable instructions associated with various CT data acquisition modes. The one or more processors 108 can receive an indication a CT data acquisition mode from the operator console 112, and execute the corresponding executable instructions.

The one or more processors 108 can receive digitized X-ray data (e.g., X-ray projections) from the control system 106 or the X-ray detector array assembly 122 and perform image reconstruction according to methods described in the present disclosure. The one or more processors 108 can cause the reconstructed CT images to be displayed on the display device 110 or stored on the storage device 114. The one or more processors 108 can include a digital a central processing unit (CPU), a microprocessor, a digital signal processor (DSP), an image processor, an application-specific instruction set processor (ASIP), a graphics processing unit (GPU), a multicore processor, or a combination thereof.

The one or more processors 108 can receive commands and/or scanning parameters from an operator via the operator console 112. The operator console 112 can include keyboard, a touch screen, a pedal, other manually operable controls, or a combination thereof. The display device 110 can include a display screen for displaying CT images and/or other data to the operator of the CT scanner 102 (or the physician performing the interventional procedure). While FIG. 1 shows the CT scanner 102 to include a single X-ray source assembly 120 and a single X-ray detector array assembly 122, the CT scanner 102 can include two or more X-ray source-detector pairs arranged at offset angle(s) with respect to one another. In such a setup, two or more projections can be acquired simultaneously by the two or more X-ray source-detector pairs.

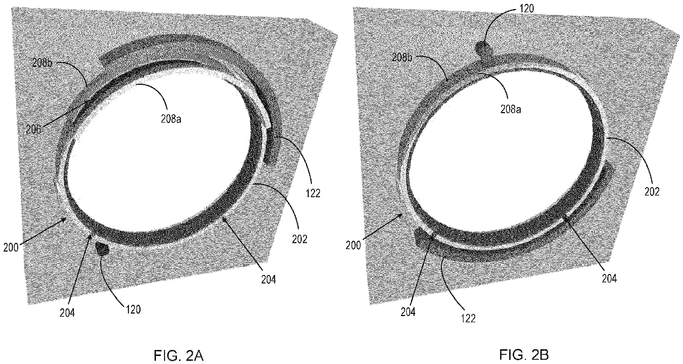

Referring to FIGS. 2A and 2B, diagrams illustrating an example X-ray filter 200 are shown, according to inventive concepts of this disclosure. As shown in FIG. 1, the X-ray filter 200 can be positioned around the CT bore 116 similar to X-ray filter 124. The X-ray filter 200 can include a curved structure 202 formed of a metal (or other material) capable of blocking X-rays emitted by the X-ray source assembly (or X-ray source) 120 from entering the CT bore 116. The metal (or material) capable of blocking X-rays can include lead, tungsten or other relatively dense material. The curved structure 202 can include two or more apertures 204 to enable X-ray beams to enter the CT bore 116 for specific viewing angles. Each aperture 204 can allow X-rays emitted by the X-ray source 120 to enter the CT bore 116 when the X-ray source 120 is aligned with the aperture 204. The X-ray filter 200 can include an opening (or gap) 206, arranged opposite to the two or more apertures 204 across the CT bore 116. The opening (or gap) 206 can be wider (or having a larger breadth) than the apertures 204 to allow for measuring X-ray signals at the X-ray detector 122. Specifically, the opening (or gap) 206 can allow for exposing the X-ray detector array assembly (or X-ray detector) 122 to X-rays emitted by the X-ray source 120 and entering the CT bore 116 through at least one of the two or more apertures 204. Specifically, X-rays emitted by the X-ray source 120 (when the X-ray source 120 is aligned with one of the apertures 204) can enter the CT bore 116 through an aperture 204 and leave the bore 116 through the opening (or gap) 206 to impinge on at least a portion of the X-ray detector array assembly 122.

The X-ray filter 200 can include two adjustable structures 208a and 208b arranged opposite to the curved structure 202 (e.g., across the CT bore 116). The two adjustable structures 208a and 208b can be formed of a metal (or material) capable of blocking X-rays. For instance, the curved structure 202 and the two adjustable structures 208a and 208b can be formed of the same metal (or material). The adjustable structures 208a and 208b can be positioned apart from each other in mode, as shown in FIG. 2A, to form the opening 206 between them. In another mode, shown in FIG. 2B, the adjustable structures 208a and 208b can be positioned adj acent to, or against, each other, to close the opening 206 and block X-rays from penetrating the CT bore when the X-ray source 120 is facing or aligned with the adjustable structures 208a and 208b. In some implementations, the X-ray filter 200 can include a single structure or more than two adjustable structures. In some implementations, the X-ray filter 200 can have no adjustable structure, in which case the whole region to which the curved structure 202 does not extend can form the opening (or gap) 206 (similar to the opening or gap 126 in FIG. 1).

The curved structure 202 and the two adjustable structures 208a and 208b can form together a ring (or another form of a closed loop). For instance, the ring can have an outer diameter equal to the size of the CT bore 116, for example, to allow the ring to be placed against the inner wall of the CT bore 116. As an illustrative example, the ring to be used in the General Electric (GE) scanner Discover HD750 can have an outer diameter equal to 70 cm. The width of the curved structure (or ring) can depend on the size of the X-ray beam emitted by the X-ray source assembly 120. Specifically, the curved structure (or ring) should be wide enough to block X-rays on the side of the X-ray source 120 from entering the CT bore 116. For example, the minimum thickness of the curved structure (or the ring) can be selected (or defined) to filter at least 99.9% of the incoming X-ray beam when the X-ray source is facing (or aligned with) the X-ray blocking region (not the apertures) of the curved structure (or the ring). For the Discover HD750 CT scanner, the minimum thickness can be equal to 1.31 mm. In simulations assuming a tungsten-anode spectrum, prefiltered by 2 mm Al, the minimum thickness was found to be 1.69 mm for 120 kV and 1.73 mm for 140 kV.

Having two or more apertures 204 can allow for generating at least two CT images associated with at least respective projection angles, per gantry rotation. Each CT image can be associated with, or generated based on projections acquired through, a respective apertures 204 of the two or more apertures 204. One of the challenges is how to design and/or build an aperture based X-ray filter system that can be integrated in an existing CT scanner without modifying the CT scanner. For instance, the distance between the X-ray source 120 (or the focal point) and the edge of the CT bore 116 is an important factor in defining or determining the breadth of each aperture 204. Such distance, for example, is approximately 19 cm for the Discovery HD750 CT scanner. In the same scanner, for an X-ray beam that covers the complete width (or breadth) of the X-ray detector array assembly 122 to enter the field of view through one of the apertures 204, the aperture 204 has to be at least 196 mm wide, or forming at least a 24° angular sector at the center of the CT bore 116. For the same X-ray beam to impinge on the whole X-ray detector array assembly 122, the opening 206 form at least a 110° angular sector at the center of the CT bore 116.

Employing two apertures where each forms angular sector that is 90° implies that only 31% of the circumference of the CT bore 116 (in the Discovery HD750 CT scanner) would be covered by the X-ray blocking metal or material. In the case where apertures forms an angular sector that is 45° wide, only 44% of the circumference of the CT bore 116 would be covered by the X-ray blocking metal or material. As such, there is a tradeoff between the desire to reduce the radiation dose for the patient and medical staff (which calls for a larger portion of the circumference of the CT bore 116 to be covered with the X-ray blocking metal or material of the X-ray filter 200) and the desire to have as wide a field view as possible (e.g., have each X-ray beam impinge on the whole X-ray detector array assembly 122). To achieve significant radiation dose reduction while allowing for accurate and reliable reconstruction of the anatomical region of interest and the interventional device, the apertures 204 can be designed to have a breadth (or width) that is too small to allow for full (or complete) excitation of (or exposure to) the X-ray detector array assembly 122, but large enough to allow for the acquisition of multiple projection images through each aperture 204. Each projection image can impinge on a different region of the X-ray detector array assembly 122. For example, for the Discovery HD750 CT scanner, the width or breadth of each aperture 204 can equal to 1.5 mm (instead of 196 mm). As such each aperture 204 can only allow for exposure of about 0.8% of the X-ray detector array assembly 122 (e.g., along the width of the detector) to the X-ray beam when the X-ray source 120 is aligned with or facing the center of the aperture. However, considering multiple positions of the X-ray source near the aperture 204, a different set of detector columns (of the X-ray detector array assembly 122) can be excited by, or exposed, to the X-ray beam emitted from each X-ray source position. The one or more processors 108 can combine the data (or projection images) associated with various X-ray source positions to create a virtual CT projection image that can be associated with the fully X-ray detector array. The virtual focal point associated with the virtual CT projection image can be associated with the X-ray source position facing the center of the aperture 204.

In general, the angular range $\theta$ representing the exposure of the X-ray detector assembly to a given X-ray beam can be described as:

$$\theta = 2 \cdot \sin^{-1} \frac{w_a \cdot \frac{d_{sd}}{d_{sa}}}{2 \cdot d_{id}}.$$

The parameter $w_a$ represents the width of the aperture, $d_{sd}$ represents the distance between the X-ray source assembly 120 and the X-ray detector array assembly 122, $d_{sa}$ represents the distance between the X-ray source assembly 120 and the aperture 204, and $d_{id}$ represents the distance between the isocenter (e.g., the center of the CT bore 116) and the X-ray detector array assembly 122. The percentage (or ratio) of the X-ray detector array assembly 122 that is exposed to the X-ray beam can be formulated as:

$$ratio = \theta \cdot \frac{d_{sd}}{2 \cdot n_c \cdot w_p}.$$

The parameter $n_c$ represents the number of columns of the X-ray detector array assembly 122, and $w_p$ represents the width of a single pixel. The control system 106 or the one or more processors 108 can determine based on the percentage (or ratio) of the X-ray detector array assembly 122 that is exposed to the X-ray beam when to use actual projection images (recorded by the X-ray detector array assembly 122) and when to use virtual CT projection images (constructed by combining two or more X-ray projection images recorded by the X-ray detector array assembly 122). For example, the control system 106 or the one or more processors 108 can decide to use virtual CT projection images, instead of actual projection images, if the exposure ratio is less than 10% or other predefined percentage value.

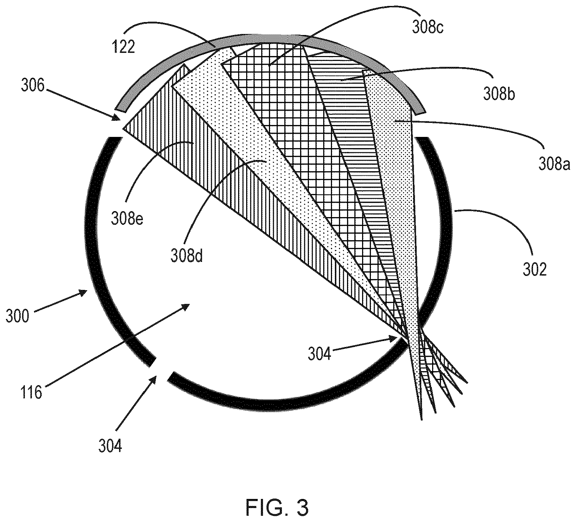

FIG. 3 shows an example illustration of multiple partial exposures of the X-ray detector array assembly 122 to X-ray beams passing through a single aperture, according to inventive concepts of this disclosure. An X-ray filter 300 can include a curved structure 302 formed of a metal (or material) capable of blocking X-rays and including a pair of apertures 204. The curved structure 302 can include a relatively wide opening (also referred to as gap or slit) 306 (e.g., wider or larger than the aperture 304) for exposing X-rays that entered the CT bore 116 to the X-ray detector array assembly 122. The slit 306 can be viewed, for example, as a missing portion in the curved structure 302 to form a ring. The X-ray filter 300 can be viewed as another version of the X-ray filter 200 of FIGS. 2A and 2B, but without the adjustable structures 208a and 208b. The concept of generating multiple projection images associated with multiple partial exposures of the X-ray detector array assembly 122 to X-ray beams passing through a single aperture 304 can be applied in the presence or absence of the adjustable structures.

As shown in FIG. 3, the X-ray source 120 (not shown in FIG. 3) can emit a plurality of X-ray beams 308a-308e from multiple positions through the aperture 304, as the X-ray source assembly 120 rotates around the CT bore 116. Each of the X-ray beams 308a-308e can imping on a respective portion (e.g., a respective number of columns) of the X-ray detector array assembly 122. The control system 106 can generate a separate projection image for each of the X-ray beams 306a-306e, for example, based on corresponding signals obtained from the X-ray detector array assembly 122. The one or more processors 108 can generate a composite CT image using the projection images corresponding to the X-ray beams 308a-308e. For instance, for each image column (or row) corresponding to a column of the X-ray detector array assembly 122, the one or more processors 108 can select the projection image, from the multiple projection images, associated with the best projection angle (or associated with the X-ray beam hat best impinges on the detector column) to retrieve the intensity values for that image column (or row). The one or more processors 108 may use a weighting approach to combine the projection images corresponding to the X-ray beams 306a-306e and form the composite CT image that can reflect intensities from all detector elements or columns.

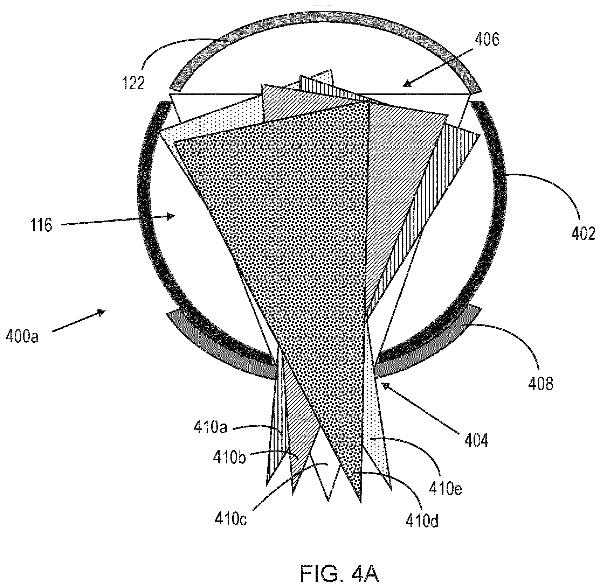

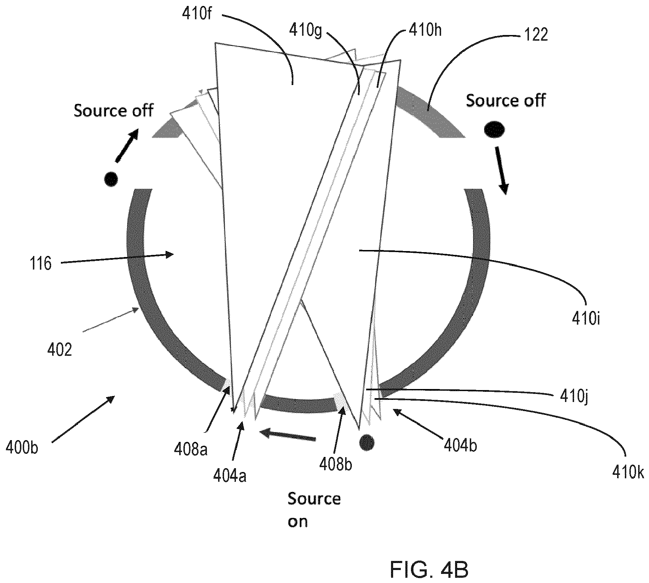

FIGS. 4A and 4B show other example X-ray filters 400a and 400b, according to inventive concepts of this disclosure. Referring to FIG. 4A, the X-ray filter 400a can include curved structure 402 formed of a metal or material, such as lead or tungsten, capable of blocking X-rays, and including an aperture 404 for allowing X-ray beams emitted by the X-ray source 120 to enter the CT bore 116 when the X-ray source 120 is facing or aligned with the aperture 404. The curved structure 402 can be viewed as a partial ring with an opening or gap 406 exposing the X-ray detector array assembly 122 to X-ray beams that enter the CT bore 116 through the aperture 404. The X-ray filter 400 can include a layer (or a structure made) of copper 408 covering the aperture 404. The layer of copper 408 can be placed against an outer surface, or an inner surface, of the curved structure 402 in a way to cover the aperture 404. The layer of copper 408 can be placed in, or arranged to fill, the aperture 404.

The aperture 404 can be, for example, at least wide enough to allow complete excitation of the X-ray detector array assembly 122 when the X-ray source assembly 120 is aligned with the center of the gap 404. Such width or breadth leads to significant exposure to radiation by the patient and medical staff performing the interventional procedure. The use of the layer of copper 408 can attenuate the amount of radiation that enters the CT bore 116 through the source aperture 404 by about a factor of 10. In fact, the layer of copper acts, or can be viewed, as an additional X-ray filter that reduces the amount of radiation entering the CT bore 116. Also, the radiation (or amperage) can be reduced from 20 mA to 10mA. In addition, the curved structure 402 can introduce a a reduction in radiation, for example, by a factor of 3 (e.g., depending on the breadth of the source aperture 404 compared to the total breadth or size of the curved structure 402). In total, the radiation can be reduced, for example, by a factor of 60 ($2 \times 10 \times 3$).

As the X-ray source 120 rotates around the CT bore 116 near the aperture 404, a plurality of X-ray beams, e.g., X-ray beams 410a-410e (also referred to herein individually or in combination as X-ray beam(s) 410), can impinge on the X-ray detector array assembly 122, and result in a corresponding plurality of projection images generated by the X-ray detector array assembly 122 or the control system 106. Note that the number of X-ray beams passing through the aperture 404, and resulting in a corresponding number of projection images, can be greater than or less than five. The use of X-ray beams 410a-410e is just for illustrative purposes and should not be limiting. Even if the aperture 404 may be relatively wide, not all the X-ray beams 410a-410e may impinge on all the detection elements of the X-ray detector array assembly 122.

In some implementations, the source aperture 404 may be large enough such that two or more projection images associated with two or more X-ray beams 410 impinge on a relatively large portion (e.g., larger than or equal to 10%, 15%, 20% or other predefined value) of the X-ray detector array assembly 122. The two or more projection images can be associated with different projection angles and can cover a field view that includes, for example, the tip and a significant portion of the interventional device. The control system 106 or the one processors 108 can use such projection images directly to reconstruct a 2-D or 3D image of the interventional device. In some implementations, the control system 106 or the one processors 108 can use the projection images (or projection signals) provided by the X-ray detector array assembly 122 (and associated with X-ray beams 410) to generate at least two virtual CT images associated with at least two distinct focal or projection angles. Each virtual CT image can represent attenuation values recorded throughout the whole X-ray detector array assembly 122. For example, the control system 106 or the one processors 108 can generate a first virtual CT image using projection images associated with the X-ray beams 410a-410c, and a second virtual CT image using projection images associated with the X-ray beams 410c-410e.

Referring the FIG. 4B, the X-ray filter 400b can be similar to X-ray filter 400a, except that curved structure 402 can two (or more) source apertures 404 and 404b. The source apertures 404a and 404b can be filled with copper filters (or layers) 408a and 408b, respectively. In some implementations, the X-ray filter 400b can include a single copper filter (or layer) 408 that covers both source apertures 404a and 404b. The source apertures 404a and 404b (or the respective centers) can be separated by an angle, for example, between 45° and 90°. The source apertures 404a and 404b can have a breadth or width smaller than the breadth or width of the source aperture 404 in FIG. 4A.

As the X-ray source 120 rotates around the CT bore 116 near the aperture 404a or 404b, a plurality of X-ray beams can be emitted through that aperture. For example, X-ray beams 410f-410g can enter the CT bore 116 through aperture 404a and X-ray beams 410i-410k can enter the CT bore 116 through aperture 404b. Note that the number of X-ray beams passing through each of the apertures 404a and 404b, and resulting in a corresponding number of projection images, can be greater than or less than three. The use of X-ray beams 410f-410g and 410i-410k is just for illustrative purposes and should not be limiting. The control system 106 or the one processors 108 can combine or integrate the projection images, or projection signals, corresponding to consecutive X-ray beams 410f-410g to generate a first virtual or composite CT image for the source aperture 404a, and can combine or integrate the projection images, or projection signals, corresponding to consecutive X-ray beams 410i-410k to generate a second virtual or composite CT image for the source aperture 404b. Each virtual CT image can represent attenuation values recorded throughout the whole X-ray detector array assembly 122. The projection angles for the first and second virtual or composite CT images can be separated, for example, by an angle between 45° and 90°.

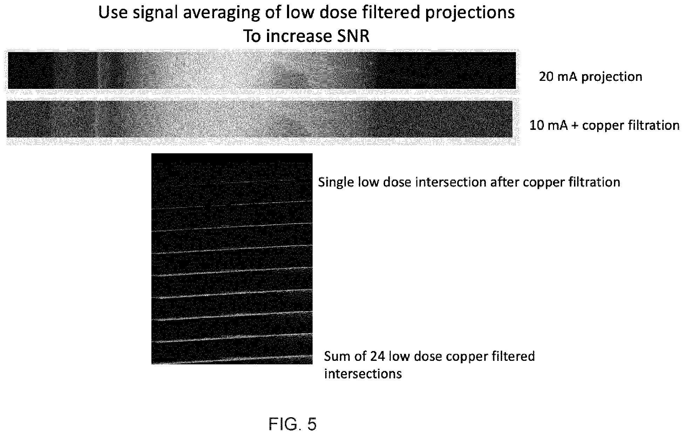

When using copper layer(s) or filter(s) 408, or 408a and 408b, combining or integrating multiple consecutive projection images can lead to substantial increase in the signal to noise ratio (SNR) of the resulting virtual or composite CT images. FIG. 5 shows simulation results illustrating the increase in SNR when integrating or combining multiple projection images. In fact, due to the significant reduction in radiation dose (e.g., by a factor of 60 as discussed above with regard to FIG. 4A) when using X-ray filters employing, for example, lead and copper filters as shown in FIGS. 4A and 4B, the SNR of the actual projection images may be relatively low. By integrating or combining a plurality of consecutive actual projection images, the SNR of composite CT images can be substantially increased, as shown in FIG. 5. While FIG. 5 indicates the integration or combination of 24 projection images, the current disclosure contemplates the integration or combination of any number of available projection images.

When designing the X-ray 400 filter to have a single aperture 404, the angular separation between the focal or projection angles of constructed virtual CT images can be relatively small, for example, compared to using multiple apertures. Specifically, in a single aperture design, such angular separation depends on the width or breadth of the aperture 404. According to some implementations, the X-ray filter 400 can include multiple apertures that are covered by one or more copper structures. In such case, the apertures can have a relative narrower breadth or width (e.g., as described with regard to FIGS. 2A, 2B and 3). The control system 106 or the one or more processors 108 can generate or construct a separate virtual CT image for each aperture, as described above with regard to FIG. 3.

Referring to FIGS. 1-4, designing the X-ray filter, e.g., X-ray filter 124, 300 or 400, to have a single relatively large (or wide) slit or opening, e.g., opening 126, 306 or 406, without adjustable structures allows for a relatively simpler design of the X-ray filter 400. Using adjustable structures, e.g., adjustable structures 208a and 208b in FIGS. 2A and 2B, calls for a mechanism to control and move the adjustable structure. Specifically, when the X-ray source 120 is aligned with the adjustable structures 208a and 208b, the controlling mechanism can cause the adjustable structures to move, e.g., toward each other, to close the opening 206 and block X-rays emitted by the X-ray source 120 from entering the CT bore 116. When no adjustable structures are used, as is the case for X-ray filters 124, 300 and 400, the goal of reducing radiation exposure for the patient and medical staff calls for preventing or avoiding X-rays from entering the CT bore 116 from the opening or slit 126, 306 or 406. One approach to avoid X-rays from entering the CT bore 16 from the opening or slit 126, 306 or 406 is to trigger, during each gantry revolution, a short scan that goes on while the X-ray source 120 is aligned, or facing, the curved structure 302 or 402 and stops when the X-ray source 120 is aligned, or facing, the opening or slit 126, 306 or 406. According to such short scan, the X-ray source 120 is actuated when it is aligned, or facing, the curved structure 302 or 402, and is deactivated (or switched off) when it is aligned, or facing, the opening or slit 126, 306 or 406.

The control system 106 or the one or more processors 108 can use audio signals recorded by the microphones 128 to control the adjustable apertures 108a and 108b of FIGS. 2A and 2B, or to control short scans when the X-ray filter 124, 300 or 400 includes an opening or slit 126, 306 or 406 with no structure(s) to adaptively cover the opening or slit 126, 306 or 406. The microphones 128 can include a plurality (e.g., four) piezo contact microphones placed at different positions or angles around the CT bore or the rotation path 20 of the X-ray source assembly 120 and/or the X-ray detector array assembly 122.

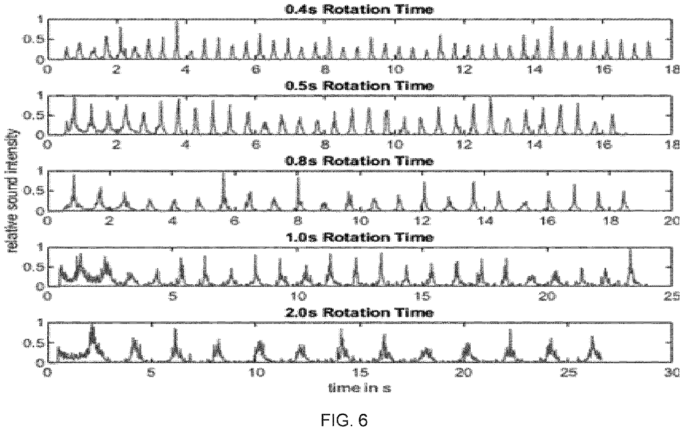

FIG. 6 shows plots illustrating audio signals recorded by a piezo microphone for various gantry revolution speeds. The piezo microphone was attached to the CT bore 116 at approximately 45 degrees from the axis 30. Various audio signals were recorded by the piezo microphone for various gantry rotation speeds where the duration of a gantry rotation is, respectively, 0.4s, 0.5s, 0.8s, 1.0s, and 2.0s. The temporal resolution of the audio signals is 44 to 100 Hz. The temporal resolution of the power spectra of the recorded audio signals is 0.0256 s. The power spectrum of the audio signal for each gantry speed is averaged over the frequency range between 5.2 and 6.5 kHz to determine the intensity of these frequencies. The plots in FIG. 6 show that a single peak in the sound level can be observed for every gantry rotation. time the X-ray detector array assembly 122 passes by the microphone The peak is correlated to the gantry position and therefore to positions of both the X-ray detector assembly 122 and X-ray source assembly 120. The first peak of the autocorrelation function was used to determine the time shift as an estimate of the gantry rotation time based on auditory tracking. All estimated rotation times were within 0.4% of the theoretical gantry rotation times.

Using the recorded audio signals, knowledge of the direction of rotation of the gantry 118 and/or the positions of the microphones 128, the control system 106 or the one or more processors 108 can track or determine the position of the X-ray source 120, or the X-ray detector array assembly 122, in real time (e.g., with a temporal resolution similar to the temporal resolution of the recorded audio signals) as the gantry rotates around the CT bore 116. The control system 106 or the one or more processors 108 can employ the determined real time position of the X-ray source 120 or the X-ray detector array assembly 122 to control the positions of the adjustable structures 208a and 208b of X-ray filter 200, or to control short scans when using an X-ray filter with un-coverable opening or slit, such as X-ray filter 124, 300 or 400.

For instance, when detecting that the X-ray source 120 is close to and moving towards the adjustable structures 208a and 208b (or any equivalent adjustable structure(s)), the control system 106 or the one or more processors 108 can cause, or trigger, the adjustable structures 208a and 208b (or any equivalent adjustable structure(s)) to move toward each other to close the opening 206 before the X-ray source 120 reaches the adjustable structures 208a and 208b. As such, the opening 206 can be closed (as shown in FIG. 2B) and the structures 208a and 208b can block X-rays emitted by the X-ray source from entering the CT bore 116.

The adjustable structures 208a and 208b can be viewed as a moving lead (or other X-ray blocking material) cover. The cover can be mounted, for example, on a rail system and can be moved in and out of the field of view in the direction perpendicular to the imaging plane using a solenoid to toggle between the two positions. The total displacement of each of the adjustable structures 208a and 208b, when moving from one position to the other, can be equal to at least half width (or diameter) of the X-ray beam in the Z-dimension. As such, the detector opening 206 can have a breadth or width in the Z-dimension at least equal to the width of the X-ray beam. The control system 106 or the one or more processors 108 can cause displacement of the adjustable structures 208a and 208b from one position to another, for example, by triggering or actuating a mechanical system, e.g., a motor that causes motion of the adjustable structures 208a and 208b. This actuation of the mechanical system signal can trigger the opening and closing of the detector cover (or the opening 206).

The control system 106 or the one or more processors 108 can apply short scans in CT scanners with a prospectively gated cardiac mode. Such CT scanners allow for an electrocardiography (ECG) signal to be provided as input to the CT scanner, and the CT scanner 102 can actuate the X-ray source 120 for a time period defined by characteristics of the ECG signal. To control the short scan for each gantry revolution, the control system 106 or the one or more processors 108 can employ an artificial ECG signal. The ECG signal can be created or constructed to cause actuation of the X-ray source 120 during the time period (of each gantry rotation) when the X-ray source 120 is facing or aligned with the curved structure 302 or 402, for example, by controlling the time lapse between R waves in the ECG signal. When detecting that the X-ray source 120 passed the microphone associated with the start of the curved structure 302 or 402 moving away from the opening or slit 126, 306 or 406, the control system 106 or the one or more processors 108 can feed the ECG signal to the CT scanner 102 as input. In response the CT scanner 102 can actuate the X-ray source 120 for a time period defined by the artificial ECG signal. Based on the characteristics of the artificial ECG signal, the X-ray source 120 can be deactivated before the X-ray source reach the opening or slit 126, 306 or 406. When employing short scans, the detector opening 126, 306 or 406 can limited to 360° minus the angular range of the short scan.

When using any of the X-ray filters described above with regard to FIGS. 1-4, the control system 106 or the one or more processors 108 can generate at least two CT images associated with at least two separate focal or projection angles for each gantry rotation. Prior to starting the interventional procedure, the CT scanner 102 can acquire a full dose scan of the anatomical region. The one or more processors 108 (or the CT scanner 102) can generate a three dimensional (3D) image of the anatomical region using the projection images acquired during the full dose scan. The one or more processors 108 (or the CT scanner 102) can determine a position and/or orientation of the interventional device within a 3D space associated with the anatomical region using the at least two CT images generated or acquired in each gantry rotation during the interventional procedure.

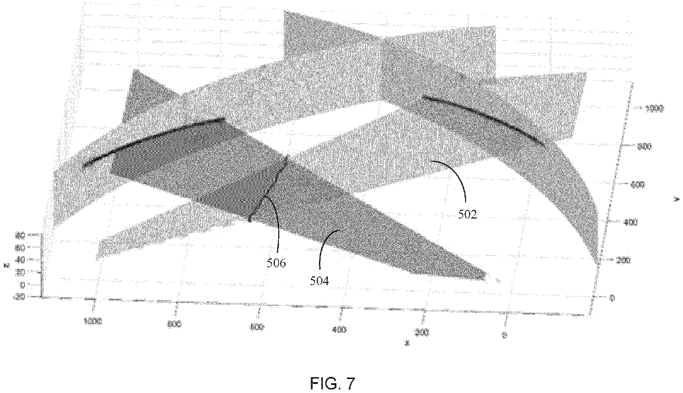

The one or more processors 108 can reconstruct, e.g., for each gantry rotation, a 2D or 3D image of the interventional device from the at least two CT images generated for that gantry rotation. The construction of 2D or 3D image of the interventional device can include segmenting the interventional device in the CT images, for example, using a line enhancement filter. The line enhancement filter uses an approximation of the local second derivative in different directions to enhance curvilinear structures. The usually highly attenuating material of the interventional device can cause high intensity signals in the filtered image. The one or more processors 108 can apply a threshold to binarize such signals. The one or more processors 108 can extract the centerline of the interventional device, for example, using topology preserving thinning. The one or more processors 108 can extract pairs of corresponding points between the two centerlines (associated with the two projection images or virtual CT images) using, for example, epipolar geometry. The one or more processors 108 can determine a 3D point for each pair by finding the intersection between the projection rays from the respective focal point to the centerline point on the X-ray detector array assembly 122. The closest point to both rays represents the 3D point, which is connected to the previous 3D point. The final 3D centerline represents the position, orientation and/or shape of the interventional device within the anatomical region. FIG. 7 shows a diagram illustrating a method of determining the position, orientation and/or shape of the interventional device in the 3D space. The intersection of the back-projected surfaces 502 and 504 corresponds to the 3D centerline 506 of the interventional device, e.g., a needle.

The one or more processors 108 (or the CT scanner 102) can superimpose a 2D or 3D image of the interventional device on the 3D (or 2D) image of the anatomical region to generate a 3D image of both the anatomical region and the interventional device for displaying, e.g., in real time, to the physician. As the interventional procedure progresses, multiple 3D images (of the anatomical region and the interventional device) can be constructed and displayed in real time reflecting the movement of the interventional device into the anatomical region. The process of constructing the 3D images (of the anatomical region and the interventional device) is described in detail in U.S. Patent Application No. 2019/0076102 and U.S. Patent Application No. 2019/0076103.

The premise of the embodiments described in this disclosure is that i) interventional devices such as needles can be accurately reconstructed using as low as two projection (or CT) images, ii) motion compensation can be employed to allow the reconstructed interventional device to be accurately superimposed on a previously acquired CT volume that can be updated in real-time, iii) using only two projection images significantly reduces dose for patients and staff, and iv) dose reduction can be achieved using an aperture based X-ray filter in combination with existing CT scanners instead of pulsed X-rays. The aperture based X-ray filter can be a multi-view filter (e.g., including two or more apertures) or a single view filter (e.g., including a single aperture).

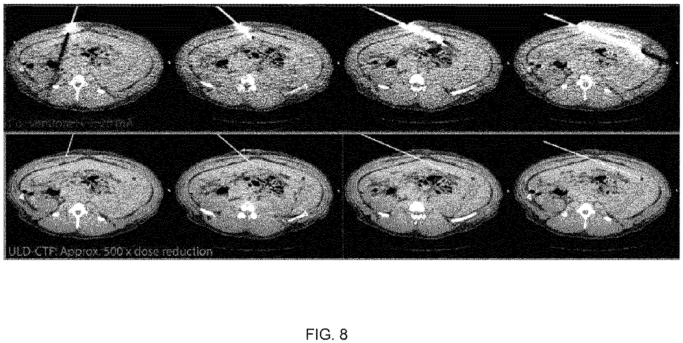

FIG. 8 shows a comparison between conventional CTF reconstructions of the interventional device and the anatomical region, and reconstructions using only two projections as discussed above. The conventional CTF reconstructions shown in the upper row of images suffer from metal artifacts. However, the reconstructions using only two projections shown in the lower row of images provide a diagnostic image quality of the target volume and the interventional device without metal artifacts.

Selection of Projection Angles

The selection of projection angles for CT images may considerably affect the accuracy of the 3D reconstructed image of the interventional device. There are several variables to consider in designing the X-ray filter including the angular separation of the two views (or corresponding apertures in a multi-aperture X-ray filter), the angle relative to the planned path of the interventional device, and the angle relative to the anatomical region. The separation angle can influence the design of the X-ray filter for image-based motion compensation techniques used to enable superimposing the reconstructed image of the interventional device on a previously acquired 3D volume of the anatomical region in intervention procedures where patient motion has to be considered. While smaller separation angles may allow for a simpler filter design, they may generally increase the condition number of the reconstruction problem. The absolute position of the angles can influence the reconstruction through overlapping highly attenuating structures such as the spine or might cause the interventional device to collapse to a single point in the projection if the angle of the needle is parallel to the viewing angle.

Continuous Acquisition

During the interventional procedure, the physician can iteratively (or continuously) change the position of the interventional device, for example, by pushing it toward a ROI or target (e.g., a tumor). Using continuous CT image acquisition allows for real time tracking of the position of the interventional device. The one or more processors 108 can reconstruct the initial position of the interventional device for the first frame assuming no motion. For subsequent frames, the one or more processors 108 can employ a 2D-3D registration technique for each single CT image, for example, using the 3D device centerline from the previous frame as initialization. The registration can be performed in two steps, first a rigid transform is estimated followed by a deformable registration to account for needle bending by minimizing a cost function. The one or more processors 108 can calculate a cost function as the 2D RMSD between the segmented centerline of the interventional device in the projection image and the forward projection of the current estimate into the 2D image plane. A regularization term can be added to the cost function to penalize large motion in the viewing direction of the current projection image. The optimization can be performed using a regular step gradient descent approach. To represent the deformable registration, the device centerline can be represented by a set of 4 control points which are connected by a cubic spline. The transformation can be represented by the individual translations of each control points.

Alternative Strategies

A possible pitfall for the implementation of the X-ray filter aperture(s) could be the softness of lead, which might make it difficult to accurately manufacture the proposed design. Alternatively, an equivalent amount of tungsten might be used instead to provide similar dose reduction. Also, the auditory tracking of the gantry position could be more difficult in other CT scanner models or for different manufacturers. In these cases, alternative methods could be used e.g. a scatter detector placed on the outside of the filter could provide reliable information on the source position and should be consistent between different CT-systems.

For the reconstruction, the robustness of the 2D segmentation represents a possible pitfall, especially in cases where highly attenuating anatomical structures are overlapping, or similar objects are in the field of view. As an alternative strategy to the proposed segmentation approach we will investigate a deep-learning approach, using a convolutional neural network such as the SegNet architecture, which can be trained based on a mix of simulated and manually annotated projection images. Ambiguities in the 3D reconstruction, represent another possible pitfall, which might occur if a straight needle is parallel to the gantry rotation plane. In this case, point correspondences cannot be determined using epipolar geometry. As an alternative strategy, we will implement a mode, where only the needle tip position is displayed to the operator. This is possible, since the tip represents a unique point on the device and the corresponding point pair can be easily determined. If other unique points are visible in the field of view such as the end point, markers, or changes in needle diameter, these points will be reconstructed as well to provide an idea of the needle direction. If a full reconstruction of the needle is required, the operator has the option to perform a conventional CT acquisition intermittently, or to change the tilt angle of the patient table. This also changes the angle of the needle and therefore resolves the ambiguities.

Reconstruction from Angular Range

The reconstruction, in the case of a single view filter (or single aperture X-ray filter), can be designed to take advantage of the larger number of projection images to overcome the decreased signal to noise ratio and the smaller separation angle between the different projections. First, the one or more processors 108 can apply the segmentation of the interventional device using line enhancement and subsequent thresholding to all projections resulting in noisy binarized images. The one or more processors 108 can apply a Hough transform for lines to determine the orientation and position of the interventional device in each 2D projection image without considering the endpoints (assuming infinitely long needle) represented by a set of points p. The one or more processors 108 can determine the 3D orientation and position by minimizing the cost function that projects the 3D line representing the current estimate into each projection image and calculating the mean squared distance from all points p to the projection of the line. The one or more processors 108 can use a second cost function to determine the endpoints of the line each represented by a single scalar value. The cost function can sample the line from start to endpoint with a fixed resolution (e.g., 0.25 mm) and project the points into the binarized 2D images. The one or more processors 108 can determine the cost by the weighted sum of all points projected onto background pixels minus all points projected onto needle pixels. The one or more processors 108 can minimize both cost functions will be minimized using the Nelder-Mead Simplex approach.

Experimental Data:

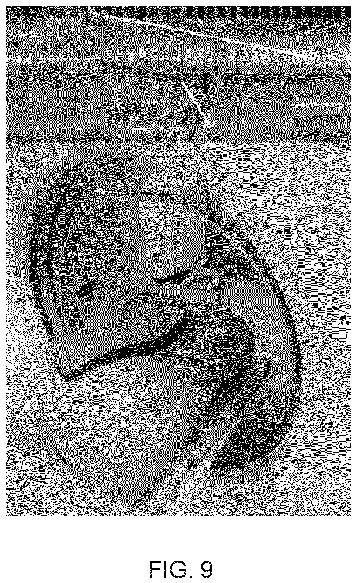

To test the multi aperture design (as shown in FIGS. 2A and 2B), a preliminary X-ray filter was built. The X-ray filter includes a 24" diameter maple wood hoop having a width equal to 40 mm. Lead strips (1.8 mm thick) were attached on the outside of the hoop leaving 1/3 (120°) uncovered as detector opening. Two small 1.5 mm apertures were placed at +/- 15° around the center point opposing the detector opening. For the preliminary studies, no moving detector cover was implemented. A preliminary study was performed, where an anthropomorphic torso phantom was placed on the patient table of the CT scanner in prone position. A biopsy needle (Chiba 18G, Cook Medical, Bloomington, IN) was placed in an oblique angle through the back of the phantom such that the needle tip located on the right side of the spine between L3 and L4. FIG. 9 shows an experimental setup including the preliminary X-ray filter. A conventional axial CT scan was performed with 120 kV and 200 mA to generate a gold standard reconstruction. The aperture system was then put in place and an axial acquisition was performed at 120 kV and 20 mA. Virtual projection images were generated by concatenating the exposed pixel columns of each projection image. The two-view reconstruction was then performed as described above and the result was compared to the commercial reconstruction of the gold standard acquisition. The reference position of the needle was extracted by thresholding the reconstruction and calculating the centerline using topology preserving thinning. The root mean squared distance (RMSD) was 0.66 mm and the tip localization error (TLE) 0.02 mm.

A preliminary single aperture system was also built using the same materials described above. The aperture was designed with a detector opening of 120° and a source aperture 160 mm opening on the opposite side. No copper filtration was used for the preliminary studies, instead additional noise was added to the digital images to reduce the signal to noise ratio. The experimental setup was the same as shown in FIG. 8. The reconstruction of the needle was performed as described in section 2b. The reference position of the needle was extracted from a conventional axial CT acquisition without the aperture in place. The RMSD was 0.76 mm and the TLE 0.03 mm.

Motion Compensation

After reconstruction of the interventional device, a 3D image of the device can be superimposed on a previously acquired 3D volume of the anatomical region to provide anatomical context. If motion occurs between the acquisition of the 3D volume of the anatomical region and the projection images for reconstruction of the interventional device, the relative position of the interventional device relative to the anatomical region might be less accurate. While it is always possible to periodically reacquire regular CT scans throughout the procedure, a method for continuous motion compensated anatomic display without extra radiation dose is desirable. Therefore, motion compensation techniques targeting three different sources of motion are considered; respiratory motion, voluntary patient motion and tissue deformation caused by the interventional device.

Model-Based Respiratory Motion Compensation

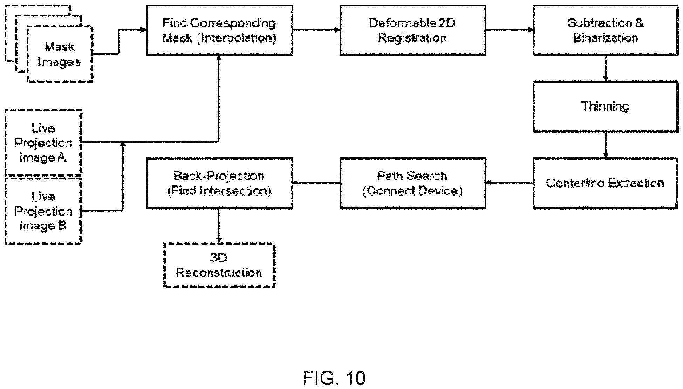

A model-based motion compensation technique can be based on two target volumes acquired at maximum expiration and inspiration respectively. The two volumes are acquired prior to device insertion and reconstructed using conventional filtered back projection. A deformable 3D registration technique (diffeomorphic demons) will be used to estimate a vector field, which describes the deformation between inspiration and expiration. This vector field can be used as respiratory motion model and can be scaled by a single parameter to simulate different respiratory states between inspiration and expiration by transforming the end expiration or inspiration target volume using the scaled vector field. Using parameters smaller than zero or larger than one would enable respiratory states that exceed the inspiration and expiration states of the target volumes. The scaling parameter representing the current respiratory state is estimated based on the two projection images used to reconstruct the device. Using the estimated parameter of the previous frame as an initial approximation, the volume is transformed and forward projected and compared to both projection images. Similarity measures can be based on Mattes mutual information and the mean squared error of the gradient magnitudes. The similarity measure can be minimized using the Nelder-Mead simplex approach. An overview of a model-based motion compensation system is shown in FIG. 10. To enable more flexible respiratory motion, models with multiple spatially distributed scaling parameters can be considered. Each parameter corresponds to a 3D point (node), where the scaling factor for points between the nodes can be calculated using linear interpolation. Different numbers of parameters will be investigated up to the point where no improvement in accuracy can be achieved by adding more parameters.

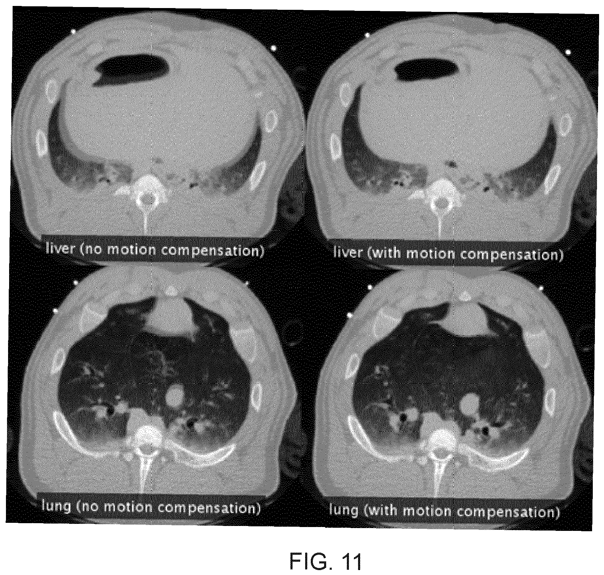

A pilot study was performed on a single female pig to investigate the feasibility of the proposed respiratory motion compensation technique. Two needle placement procedures were performed in the lung and liver respectively, using conventional CT imaging. Each sequence contains a target acquisition prior to device placement followed by eleven acquisitions with the needle in place. After each acquisition the needle was advanced to a different location and orientation. The lung sequence was acquired at both 10 mA and 100 mA, while the liver sequence was acquired at 20 mA and 200 mA. For each image acquisition the ventilation was stopped at a random respiratory state. A breathing bag was used to manually keep the pressure constant. The reference motion was determined by applying the diffeomorphic demons approach to perform deformable registration between the target volume and the conventional reconstruction of each acquisition. The estimated vector field was used as the gold standard. The motion compensation technique described above was applied using only two projection images at fixed projection angles of -40 and 50 degree to the anteroposterior axis. The estimated transform was then compared to the reference at ten points of interest along organ boundaries. The registration error for the 200 mA and 100 mA acquisitions was 1.52 ± 1.08 mm and 2.12 ± 1.48 mm for the 20 mA and 10 mA acquisitions. A comparison of the results with and without motion compensation is shown in FIG. 11, where the 3D volume of the anatomical region is overlaid with the conventional reconstruction of the interventional device at a random respiratory state.

Fiducial-Based Motion Compensation for Voluntary Patient Motion

A practical fiducial based motion compensation technique can be used to target voluntary patient motion during the procedure. External fiducials can be attached to the skin of the patient prior to acquiring the target volume. The fiducials are then extracted from the target volume using global threshold-based segmentation. During the procedure, the fiducial positions can be extracted from the projection images used to reconstruct the device. This can be done using threshold-based segmentation. The centers of the fiducials can then be back-projected into the 3D space from both images and the intersections of the projection rays represent the 3D fiducial positions. A rigid transformation can then estimated to align the 3D fiducial positions with the locations extracted from the target volume. At least two fiducials are required to calculate a rigid transform analytically. Additional fiducials can be considered to increase accuracy or allow affine transforms. Since external fiducials are used for this technique this approach might be most suitable for voluntary patient motion.

Cumulative Reconstruction for Tissue Deformation

A cumulative reconstruction technique can be employed to address small tissue deformations near the needle. The projection images used to reconstruct the device can be collected over multiple frames. A compressed sensing reconstruction on a small set of projection images is then performed using the target volume as constraint volume. The initial subset of projection images can be created from the target volume acquisition. Each time a new projection image is acquired it can be added to the subset, while the oldest projection image will be removed. During the reconstruction, the needle (or interventional device) can be masked out in the projection images to avoid artifacts due to changing needle position. In each gantry rotation different projection angles can be be used to increase the number of distinct projection angles available for reconstruction. The total number of projection images in the subset can determine the quality of the reconstruction. However, using a larger subset can also increase the risk of motion between the projection images, which might cause artifacts in the reconstruction. Different subset sizes can be considered. A deformable 3D registration can be used to align the compressed sensing reconstruction for each frame with the original target volume. This can allow transforming the diagnostic quality target volume before superimposing the reconstructed device. Since this technique uses projection images acquired over a longer period of time (several seconds) changes in the volume might not immediately show up in the reconstruction, instead multiple projection images might be required after motion occurs until it is visible. Therefore, this technique can be more suited for slowly changing motion such as tissue deformation caused by the device itself. Besides the accuracy of this motion compensation technique the response time can also be evaluated.

Image noise could be a major challenge for the proposed model-based respiratory motion compensation technique as well as the cumulative reconstruction, since the accuracy of the estimated scaling parameters and the 3D registration respectively could be reduced. Noise reduction techniques such as Gaussian and bilateral filter can be applied prior to calculating the similarity metric or after compressed sensing reconstruction to investigate if the motion compensation accuracy can be improved. Additionally, the results can be compared for images acquired at 200 mA and 20 mA.

While, the preliminary experimental data seems promising in terms of determining patient motion and the complexity of the proposed model(s) can be easily increased by adding additional parameters, another alternative strategy may be employed or considered. A planning CT acquisition can be performed prior to needle insertion. During the procedure, two projection images will be acquired per frame to perform the ultra-low dose device reconstruction as described in aim 1. The result can be superimposed on the planning CT. However, to reduce the influence of motion, a low dose conventional CT can be acquired intermittently and a 3D-3D registration between planning CT and low dose CCT performed to update the anatomical information. In clinical practice this could be used for a combination between step-and-shoot and continuous imaging. The patient would be instructed to hold the breath for a certain period of time (e.g. 10 s), the low dose CCT acquisition will then be applied to update the planning CT followed by a series of ULD-CTF acquisitions during which the needle is advanced under real-time guidance. The patient can then continue to breath normally until the next sequence. These steps would be continued until the needle reaches the target location. Despite the intermittent low dose CCT acquisitions, this technique would still enable considerable dose reduction compared to conventional CTF or CCT guidance.

The respiratory and fiducial based motion compensation techniques described above can be evaluated based on digital simulations generated based on the XCAT suite. The XCAT suite provides realistic 4D CT phantoms of an average male and female person including all major organs and vessels as well as sophisticated respiratory and cardiac motion models. The XCAT suite will be used to create voxelized CT volumes at different respiratory and cardiac states. Additional patient motion will be simulated using rigid transforms. The virtual needle advancements and the generation of the projection images can be performed as described above. Each time frame can be simulated with a random respiratory state. A planning CT without virtual needle can be simulated at end expiration with 100 mA for targets in the lung and 200 mA for all other cases. Additionally, a low dose acquisition at end inspiration can be simulated 10 mA or 20 mA respectively for the respiratory motion compensation. Virtual fiducials can be manually placed in the XCAT phantom corresponding to positions on the skin of a patient. The dimensions of the fiducials used in the animal experiments can be measured and the attenuation determined in CT acquisitions to create realistic representations in the phantom. For the digital simulations, the ground truth of the respiratory and voluntary patient motion are known and can be directly compared to the transformations estimated by the motion compensation techniques.

An ex-vivo study using 10 excised cow livers can be performed to validate the motion compensation for tissue deformation caused by the needle. Therefore, the liver can be placed in the field of view and a planning CT is acquired without a needle. A sequence of CCT acquisitions with 10 mA (120 kV) can then acquired with small needle advancements in between acquisitions. Additionally, a dual energy acquisition with metal artifact reduction (DE- MAR) can be acquired for each step and is used as the gold standard. To determine the true deformation of the tissue around the needle, the needle can be first segmented in the DEMAR reconstruction using a global threshold segmentation. A deformable 3D-3D registration (diffeomorphic demons) can then be applied to register the planning CT to the DEMAR reconstruction, while ignoring all voxels containing the needle. The resulting motion vector field can be used as reference of the true motion. The same steps can be performed for the cumulative reconstruction (motion- compensation) for each frame to determine the motion vector field corresponding to the cumulative reconstruction. The resulting motion vector field can describe the estimated motion.

Finally, retrospective analysis of pilot animal studies can be performed for all motion-compensation techniques. The data can include two needle placement sequences in the lung and liver, where each frame was acquired at a random respiratory position. Each acquisition can include a full gantry rotation with 984 projection images. Full dose reconstructions with metal artifact reduction using all projection images can be created and reference (gold standard) motion vectors are extracted as described for the ex-vivo study.

The accuracy of the motion-compensation techniques can be evaluated in terms of the target registration error (TRE) at selected points of interest. Both manually selected points of interest as well as automatically selected key points (FAST points) can be used for evaluation. The FAST points can be selected based on their local neighborhood to ensure that the points are suitable for tracking. This allows more precise measurement of the registration error. The points of interest can be selected in the planning CT. For each frame of a sequence, the TRE can be calculated by looking up the transformation of each point of interest in the reference and estimated vector field and calculating the Euclidean distance.

## PREFERRED ASPECTS OF THE INVENTION

According to a first aspect of the invention there is provided an X-ray filter, comprising: a curved structure for positioning around a computed tomography (CT) bore of a CT scanner, the curved structure (i) formed of a metal capable of blocking X-rays emitted by an X-ray source of the CT scanner, and (ii) including a copper-filled aperture for allowing a portion of X-rays emitted by the X-ray source to enter the CT bore when the X-ray source is aligned with the copper-filled aperture, the copper-filled aperture having a breadth such that the portion of X-rays entering the CT bore through the aperture, when the X-ray source is aligned with a center of the aperture, excites the whole CT detector; and an opening, arranged opposite to the aperture across the CT bore, for exposing an X-ray detector of the CT scanner to the portion of X-rays emitted by the X-ray source and entering the CT bore through the aperture.

In the X-ray filter of the first aspect of the invention, the metal capable of blocking X-rays emitted by the X-ray source of the CT scanner may include lead.

The X-ray filter of the first aspect of the invention may further comprise: one or more adjustable structures for blocking X-rays emitted by the X-ray source when the X-ray source is aligned with the opening.

In the X-ray filter of the first aspect of the invention, positioning of the one or more adjustable structures may be adjustable based on a position of the X-ray source with respect to the opening.

In the X-ray filter of the first aspect of the invention, the X-ray source may be: actuated along a first portion of a revolution around the CT bore when the X-ray source is aligned with the curved structure; and deactivated along a second portion associated of the revolution around the CT bore when the X-ray source is aligned with the opening.

According to a second aspect of the invention there is provided a computed tomography (CT) scanner, comprising: a CT bore; a rotating gantry including an X-ray source and an X-ray detector positioned opposite to one another across the CT bore; one or more processors; and an X-ray filter including: a curved structure for positioning around the CT bore, the curved structure (i) formed of a metal capable of blocking X-rays emitted by the X-ray source, and (ii) including a copper-filled aperture for allowing a portion of X-rays emitted by the X-ray source to enter the CT bore when the X-ray source is aligned with the copper-filled aperture, the copper-filled aperture having a breadth such that the portion of X-rays entering the CT bore through the aperture, when the X-ray source is aligned with a center of the aperture, excites the whole CT detector; and an opening, arranged opposite to the copper-filled aperture across the CT bore for exposing the X-ray detector to the portion of X-rays emitted by the X-ray source and entering the CT bore through at least one of the two or more apertures.

In the CT scanner of the second aspect of the invention, the metal capable of blocking X-rays emitted by the X-ray source may include lead.

The CT scanner of the second aspect of the invention may further comprise: one or more adjustable structures for blocking X-rays emitted by the X-ray source when the X-ray source is aligned with the opening, the one or more adjustable structures being formed of the metal capable of blocking X-rays emitted by the X-ray source.

Such CT scanners may further comprise: one or more microphones positioned at one or more predefined positions around the CT bore for recording audio signals associated with motion of the gantry, the one or more processors configured to: monitor the position of the X-ray source with respect to the opening using the audio signals recorded by the one or more microphones; and actuate the one or more adjustable structures to adjust respective positioning based on the position of the X-ray source with respect to the opening.

Whether or not such CT scanners further comprise one or more microphones, in such CT scanners one or more adjustable structures may include a pair of structures, and the one or more processors may be configured to cause the pair of structures to: move away from each other responsive to detecting a motion of the X-ray source towards the opening; and move toward each other responsive to detecting a motion of the X-ray detector towards the opening.

In the CT scanner of the second aspect of the invention, the one or more processors may be configured to: actuate the X-ray source along a first portion of a revolution around the CT bore when the X-ray source is aligned with the curved structure; and deactivate the X-ray source along a second portion associated of the revolution around the CT bore when the X-ray source is aligned with the opening.

Such CT scanners may further comprise: one or more microphones positioned at one or more predefined positions around the CT bore for recording audio signals associated with motion of the gantry, the one or more processors configured to: monitor the position of the X-ray source using the audio signals recorded by the one or more microphones; and actuate the X-ray source based on the position of the X-ray source.

In actuating the X-ray source in such CT scanners, the one or more processors may be configured to provide an artificial electrocardiography (ECG) signal as input to the CT scanner, the CT scanner being configured to actuate the X-ray source for a time interval defined by characteristics of the ECG signal.

**Claims**

1. An X-ray filter, comprising:

   a curved structure for positioning around a computed tomography (CT) bore of a CT scanner, the curved structure formed of a metal capable of blocking X-rays emitted by an X-ray source of the CT scanner, and including:
   two or more apertures, each aperture of the two or more apertures allowing X-rays emitted by the X-ray source to enter the CT bore when the X-ray source is aligned with the aperture; and
   an opening, arranged opposite to the two or more apertures across the CT bore, for exposing an X-ray detector of the CT scanner to X-rays emitted by the X-ray source and entering the CT bore through at least one of the two or more apertures.

2. The X-ray filter of claim 1, wherein the metal capable of blocking X-rays emitted by the X-ray source of the CT scanner includes lead.

3. The X-ray filter of claim 1, wherein a breadth of each aperture of the two or more apertures is smaller than a breadth of the opening.

4. The X-ray filter of claim 1, wherein a breadth of each aperture of the two or more apertures is such that the X-rays entering the CT bore through the aperture excite only a portion of the CT detector.

5. The X-ray filter of claim 1, further comprising:
   one or more adjustable structures for blocking X-rays emitted by the X-ray source when the X-ray source is aligned with the opening.

6. The X-ray filter of claim 5, wherein positioning of the one or more adjustable structures is based on a detected position of the X-ray source with respect to the opening.

7. A computed tomography (CT) scanner, comprising:

   a CT bore;
   a rotating gantry including an X-ray source and an X-ray detector positioned opposite to one another across the CT bore;
   one or more processors; and
   an X-ray filter including:

      a curved structure for positioning around the CT bore, the curved structure (i) formed of a metal capable of blocking X-rays emitted by the X-ray source, and (ii) including two or more apertures, each aperture of the two or more apertures allowing X-rays emitted by the X-ray source to enter the CT bore when the X-ray source is aligned with the first aperture; and
      an opening, arranged opposite to the two or more apertures across the CT bore, for exposing the X-ray detector to X-rays emitted by the X-ray source and entering the CT bore through at least one of the two or more apertures.

8. The CT scanner of claim 7, wherein the metal capable of blocking X-rays emitted by the X-ray source includes lead.

9. The CT scanner of claim 7, wherein a breadth of each aperture of the two or more apertures is smaller than a breadth of the opening.

10. The CT scanner of claim 7, wherein a breadth of each aperture of the two or more apertures is such that the X-rays entering the CT bore through the aperture excite only a portion of the CT detector.

11. The CT scanner of claim 7, further comprising: one or more adjustable structures for blocking X-rays emitted by the X-ray source when the X-ray source is aligned with the opening, the one or more adjustable structures being formed of the metal capable of blocking X-rays emitted by the X-ray source.

12. The CT scanner of claim 11, further comprising:

   one or more microphones positioned at one or more predefined positions around the CT bore for recording audio signals associated with motion of the gantry, the one or more processors configured to:

   monitor the position of the X-ray source using the audio signals recorded by the one or more microphones; and actuate the one or more adjustable structures to adjust respective positioning based on the position of the X-ray source.

13. The CT scanner of claim 12, wherein one or more adjustable structures include a pair of structures, and the one or more processors are configured to cause the pair of structures to:

   move away from each other responsive to detecting a motion of the X-ray source towards the opening; and move toward each other responsive to detecting a motion of the X-ray detector towards the opening.

14. The CT scanner of claim 7, further comprising:

   one or more microphones positioned at one or more predefined positions around the CT bore for recording audio signals associated with motion of the gantry, the one or more processors configured to:

monitor the position of the X-ray source using the audio signals recorded by the one or more microphones; and actuate the X-ray source based on the position of the X-ray source.

15. The CT scanner of claim 14, wherein in actuating the X-ray source, the one or more processors are configured to provide an artificial electrocardiography (ECG) signal as input to the CT scanner, the CT scanner being configured to actuate the X-ray source for a time interval defined by characteristics of the ECG signal.

FIG. 1

FIG. 2A

FIG. 2B

FIG. 3

FIG. 4A

FIG. 4B

## Use signal averaging of low dose filtered projections
## To increase SNR

20 mA projection

10 mA + copper filtration

Single low dose intersection after copper filtration

Sum of 24 low dose copper filtered
intersections

FIG. 5

FIG. 6

FIG. 7

FIG. 8

FIG. 9

```
Mask          →    Find Corresponding   →   Deformable 2D   →   Subtraction &
Images             Mask (Interpolation)     Registration        Binarization
                          ↑                                          ↓
Live                      |                                        Thinning
Projection                |                                          ↓
image A    ──────────────┘                                   Centerline Extraction
                                                                     ↑
Live               Back-Projection    ←   Path Search      ←
Projection         (Find Intersection)    (Connect Device)
image B                   ↓
                        3D
                   Reconstruction
```

FIG. 10

28

FIG. 11

Europäisches
Patentamt

European
Patent Office

Office européen
des brevets

# EUROPEAN SEARCH REPORT

Application Number

EP 20 18 5090

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/008856 A1 (KONINKL PHILIPS NV [NL]) 21 January 2016 (2016-01-21) | 1-11 | INV. A61B6/10 A61B6/03 A61B6/00 A61B6/06 |
| A | * page 6, line 32 - page 13, line 25; figure 6 * | 12-15 | |
| X | US 2008/237473 A1 (URIBE JORGE [US] ET AL) 2 October 2008 (2008-10-02) | 1-11 | |
| A | * paragraph [0040] - paragraph [0127] * | 12-15 | |
| A | US 6 396 902 B2 (ANALOGIC CORP [US]) 28 May 2002 (2002-05-28) * column 3, line 46 - column 9, line 4 * | 1-15 | |

TECHNICAL FIELDS
SEARCHED      (IPC)

A61B
G21K

The present search report has been drawn up for all claims

| Place of search | Date of completion of the search | Examiner |
|---|---|---|
| Munich | 18 November 2020 | Hooper, Martin |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 18 5090

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2020

| Patent document cited in search report | | | Publication date | Patent family member(s) | | | Publication date |
|---|---|---|---|---|---|---|---|
| WO 2016008856 | A1 | | 21-01-2016 | CN | 106572823 | A | 19-04-2017 |
| | | | | EP | 3169240 | A1 | 24-05-2017 |
| | | | | JP | 6679563 | B2 | 15-04-2020 |
| | | | | JP | 2017524460 | A | 31-08-2017 |
| | | | | US | 2017172525 | A1 | 22-06-2017 |
| | | | | WO | 2016008856 | A1 | 21-01-2016 |
| US 2008237473 | A1 | | 02-10-2008 | NONE | | | |
| US 6396902 | B2 | | 28-05-2002 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62871803
- US 20190076102 A
- US 20190076103 A